# FASCICULE DE BREVET EUROPEEN

(11) **EP 1 229 866 B1**
(45) Date de publication et mention de la délivrance du brevet: **30.03.2005**
(21) Numéro de dépôt: 00977639.4
(22) Date de dépôt: 09.11.2000
(51) Int. Cl.: A61F 2/08

(54) **DISPOSITIF DE PROTECTION DES NERFS APRES INTERVENTION CHIRURGICALE**
VORRICHTUNG ZUM SCHÜTZEN DER NERVEN NACH EINER CHIRURGISCHEN BEHANDLUNG
DEVICE FOR PROTECTING NERVES AFTER SURGICAL PROCEDURE

(30) Priorité: 19.11.1999 FR 9914547
(43) Date de publication de la demande: 14.08.2002
(73) Titulaire: Proconcept SA, 84350 Courthezon (FR)
(72) Inventeur: DUCHE, Renaud, F-30400 Villeneuve-lès-Avignon (FR)
(74) Mandataire: Schmitt, John
(86) Numéro de dépôt international: PCT/FR2000/003117
(87) Numéro de publication internationale: WO 2001/035866

(56) Documents cités:
- GB-A- 2 324 471
- US-A- 5 104 400

## Description

La présente invention a pour objet un dispositif de protection des nerfs après intervention chirurgicale.

Le secteur technique de l'invention est le domaine de la réalisation de dispositifs chirurgicaux implantables dans le corps humain.

L'une des applications principales de l'invention est la réalisation de dispositif de protection pour le nerf médian et les tendons fléchisseurs de la main, au niveau de la zone canalaire du poignet constituant le canal carpien, et délimitée par les os de celui-ci (carpes), et le ligament annulaire antérieur correspondant.

Les personnes qui sont amenées en effet à effectuer des gestes répétitifs de la main peuvent avoir des inflammations du tissu synovial qui entoure les tendons, avec prolifération de synovite fibreuse. Les tendons se trouvent alors engainés et le volume interne du canal augmente, rendant insensibles les doigts au bout d'un certain temps par compression du nerf médian, et ne permettant plus d'effectuer un travail manuel. Une des solutions chirurgicales retenue à ce jour, et la plus efficace pour diminuer cette inflammation, est de traiter le syndrome du canal carpien en ouvrant chirurgicalement ledit ligament pour décomprimer le canal carpien et enlever la synoviale qui s'y est développée : plus de 60.000 opérations de ce type sont réalisées en France chaque année, induisant pour chaque personne, au moins deux mois d'arrêt de travail avant de pouvoir récupérer l'usage complet de leur main.

Cependant, du fait de l'ouverture du ligament annulaire transversal, dont les berges ainsi coupées restent flottantes, les tendons fléchisseurs des doigts peuvent sortir du canal carpien et le nerf médian n'est pas protégé ; le patient ne peut donc pas utiliser sa main trop rapidement et même malgré les précautions prises, on note 5% de récidive.

Pour pallier cet inconvénient, des chirurgiens utilisent différentes méthodes telle que la découpe partielle d'une des berges du ligament pour la déplacer et la raccorder à l'autre berge, au risque d'un déchirement de ce ligament qui est en effet alors affaibli.

Il n'y a pas en fait à ce jour, de réponse satisfaisante aux problèmes post-opératoires créés par l'ouverture du ligament, et cela quelle que soit la zone canalaire concernée, que ce soit au poignet, qui est le cas le plus fréquent ou ailleurs, tel qu'au coude ou à la cheville.

Selon la présente invention, une solution aux problèmes posés est un dispositif de protection des nerfs et/ou tendons, situés dans toute zone canalaire du corps humain, normalement fermée par un ligament et qui a été ouverte lors d'une intervention chirurgicale. Selon l'invention, le dispositif comprend au moins une plaque rigide ou semi-rigide, destinée à être insérée entre les bords coupés dudit ligament, après coupure effectuée lors de ladite intervention chirurgicale, et dont une face comporte une surface de glissement et l'autre face une surface d'adhérence.

Par "plaque semi-rigide", on entend que celle-ci est capable de garder une forme notamment tuilée, lorsqu'elle a été fabriquée notamment par moulage dans le cas où la plaque est réalisée en matériau synthétique. Mais il doit être entendu que la plaque peut présenter une certaine élasticité, c'est-à-dire qu'elle peut subir des déformations, notamment à l'occasion de son implantation, puis revenir à sa forme initiale.

Par "surface de glissement", on entend une surface lisse, notamment sans aspérité ou bosse, de faible coefficient de frottement, de manière à ne pas empêcher le mouvement du nerf et des tendons par rapport à ladite plaque.

Par "surface d'adhérence", on entend une surface présentant une texture et/ou une rugosité, notamment une porosité, de manière à favoriser la réhabitation de tissus biologiques notamment de ligaments sectionnés par accrochage des fibroplastes qui peuvent recoloniser ladite surface.

Pour permettre l'insertion de la plaque entre les bords coupés dudit ligament après coupure, la plaque doit présenter avantageusement une forme et des dimensions adaptées respectivement au site d'implantation c'est-à-dire à la forme de la zone canalaire et à la coupure effectuée.

En particulier, les dimensions de la plaque doivent permettre son insertion entre les bords coupés du ligament. De préférence, la longueur et la largeur correspondent avec l'ouverture du ligament et notamment la distance séparant lesdits bords après coupure, la longueur de la plaque étant donc au plus égale à celle de l'ouverture.

De préférence, la forme de ladite plaque correspond à la forme anatomique de la zone canalaire au site d'insertion. Elle peut être notamment de forme plane ou bombée.

Lorsque ladite plaque présente une forme bombée, ladite surface de glissement se trouve sur sa face concave et ladite surface d'adhérence sur sa face convexe.

Dans un mode de réalisation, ladite plaque est de forme bombée, avec au moins deux côtés curvilignes parallèles dans la direction transversale aux côtés correspondant aux bords ou berges coupés du ligament.

Pour une protection plus particulièrement adaptée à la zone canalaire du poignet, ladite plaque est de forme tuilée, c'est-à-dire en forme de calotte cylindrique avec deux côtés parallèles rectilignes dans la direction longitudinale correspondant aux bords coupés dudit ligament, et deux côtés parallèles curvilignes dans la direction transversale.

Ladite plaque peut aussi être tuilée de forme tronconique avec seulement les deux côtés curvilignes parallèles.

Pour une adaptation à d'autres zones canalaires, par exemple au niveau du coude, ladite plaque bombée doit être, non pas de forme de calotte cylindrique, mais une surface de révolution doublement courbée, c'est-à-dire avec 4 côtés curvilignes parallèles deux à deux, pour suivre le profil de la gouttière canalaire.

Dans un mode de réalisation particulier, les quatre angles de ladite plaque définissent un rectangle dont la longueur est inférieure à la taille de la coupure et le rapport largeur/longueur est de 1/ 4 à 3/ 4, de préférence 1/3 à 2/3.

Plus particulièrement, ladite plaque a une longueur de 10 à 25 mm et une largeur de 3 à 15 mm.

De même, ladite plaque est de préférence d'épaisseur quasi égale à celle dudit ligament coupé.

Ladite plaque selon l'invention est réalisée avec des biomatériaux, c'est-à-dire des matériaux implantables dans le corps humain sans risque de rejet. Les biomatériaux sont bien connus de l'homme de l'art dans le domaine de la chirurgie.

Pour pouvoir maintenir cette continuité après sa mise en place, ladite plaque 10 est d'une rigidité suffisante pour garder sa forme, notamment bombée, par elle-même. Cette rigidité peut être obtenue avec un matériau tel que du métal, pouvant constituer une partie de ladite plaque, notamment sa surface de glissement ou avec un matériau synthétique élastomère, tel que du silicone ou du polyuréthane, son épaisseur devant être alors de préférence de l'ordre de 1 à 3 mm pour conférer une rigidité suffisante à ladite plaque.

De préférence, pour obtenir les résultats souhaités, ladite plaque 10 est fabriquée en deux couches de biomatériaux de propriétés de glissement et d'adhérence différentes, assemblée entre elles et constituant chacune et respectivement la surface de glissement et la surface d'adhérence.

Les biomatériaux utilisés peuvent être résorbables, comme les polymères et copolymères polylactique et polyglycollique, en particulier le biomatériau correspondant à la surface d'adhérence 10₁ qui sera pris dans la reconstitution des tissus qui assureront la fermeture du canal carpien après réhabitation, est avantageusement un matériau résorbable.

De préférence, la rugosité Ra de la surface de glissement est inférieure à 4 microns, notamment de 0,5 à 4 microns, de préférence inférieure à 3,2 microns, de préférence encore inférieure à 0,8 microns. La rugosité Ra est connue de l'homme de l'Art : elle correspond à un écart moyen arithmétique des creux et des bosses de la surface par rapport à une ligne moyenne.

De préférence, la rugosité de la surface d'adhérence est définie par une rugosité Rt d'au moins 50 microns, de préférence au :moins 100 microns. La rugosité Rt correspond à l'écart total maximum entre le sommet des bosses et le fond des creux de la texture de la surface.

Dans un mode de réalisation avantageux, la surface de glissement est réalisée en un matériau élastomère biocompatible, notamment du polyuréthane ou du silicone.

De même, la surface d'adhérence est avantageusement réalisée par un matériau polymère biocompatible à structure microporeuse, notamment à structure fibrillaire, tel qu'un matériau en fibres de polyester. Ladite structure microporeuse comporte des pores dont la taille moyenne est de préférence de 50 à 600 microns.

De façon avantageuse, ladite plaque est constituée d'élastomère, notamment de silicone ou polyuréthane recouvert sur une face d'un treillis de fils de fibre d'un polymère biocompatible, notamment de polyester, ancré sur ladite face. On entend ici par treillis un tissu en multifilaments de fibres synthétiques à mailles ajourées.

Dans un mode de réalisation, la plaque comprend 5 à 40% en poids de matériau microporeux, notamment de polyester sous forme de treillis, et 60 à 95% en poids d'élastomère biocompatible, notamment de silicone, le total faisant 100%.

De préférence, la plaque est réalisée en matériau(x) résorbable(s).

Pour assurer le maintien de ladite plaque dans l'ouverture du ligament, celle-ci peut comprendre au moins quatre trous aptes à en assurer la suture avec les bords du ligament ou au moins quatre crochets aptes à être ancrés dans les bords du ligament.

Dans un mode de réalisation avantageux permettant le laçage de la plaque, la plaque comprend des trous obliques, c'est-à-dire dont l'axe est incliné.

La plaque peut également être solidarisée avec les bords du ligament par un moyen tel que des agrafes.

Le résultat est un nouveau dispositif de protection des nerfs et/ou tendons situés dans toute zone canalaire du corps humain, telle que celle du poignet, mais pouvant être appliqué dans toute autre zone canalaire : ce dispositif répond aux problèmes posés puisque, d'une part il maintient la continuité du ligament annulaire qui pourra ainsi cicatriser d'autant plus vite, et d'autre part il empêche, pendant cette cicatrisation, les nerfs et les tendons situés dans la zone canalaire, de sortir de celle-ci ; de plus, la face comportant la surface de glissement et qui est disposée vers la zone canalaire permet une liberté de mouvements des tendons qui s'y trouvent, alors que la face opposée correspondant à la surface d'adhérence, et disposée sur le dessus soit vers la peau, permet ainsi une réhabitation plus rapide des tissus et donc de la cicatrisation.

On obtient ainsi, grâce au dispositif de l'invention, à la fois la fermeture de la zone canalaire, la protection des nerfs et des tendons, la stabilisation des berges du ligament et la conservation du volume de la zone canalaire ; on observe ainsi une diminution importante du temps de récupération, bien inférieur aux deux mois d'arrêt nécessaires à ce jour, avec une diminution des récidives et de la douleur supportée par les patients.

D'autres caractéristiques et avantages de la présente invention apparaîtront à la lumière de la description détaillée qui va suivre.

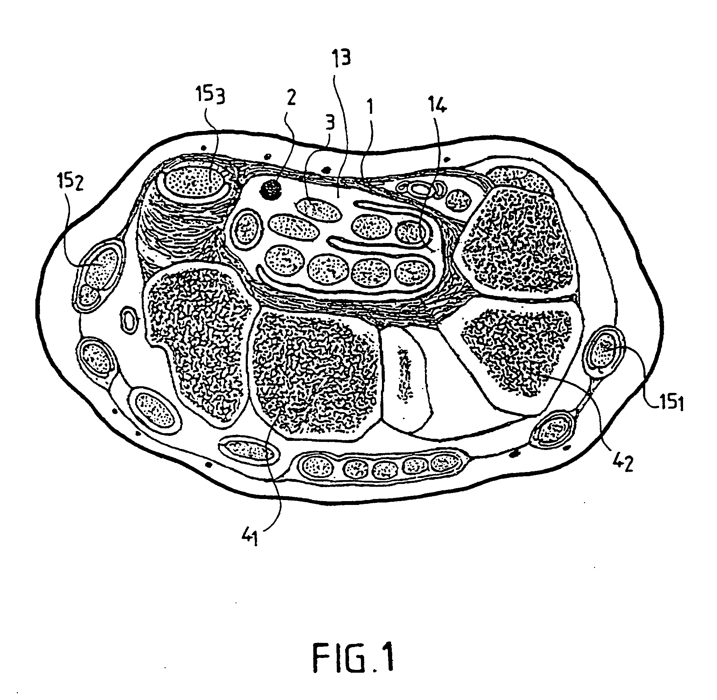

La description et les figures ci-après représentent un exemple de réalisation de l'invention, mais n'ont aucun caractère limitatif : d'autres réalisations sont possibles, dans le cadre de la portée et de l'étendue de la présente invention, en particulier pour des applications dans d'autres zones canalaires du corps humain, alors que l'exemple décrit dans la figure 1, et qui est l'application principale de la présente invention, concerne la chirurgie de la main.

La figure 1 est une vue en coupe transversale de la région du poignet.

La figure 2 est une vue en coupe transversale simplifiée d'un poignet avant une intervention chirurgicale au cours de laquelle le dispositif de protection des nerfs suivant l'invention pourra être utilisé.

La figure 3 est une vue en coupe partielle du poignet de la figure 1, après intervention chirurgicale, mais sans le dispositif suivant l'invention.

Les figures 4A et 4B représentent respectivement une vue en coupe et une vue de dessus d'un dispositif suivant l'invention.

La figure 5 est une vue en coupe partielle correspondant à celle de la figure 2, mais après la mise en place d'un dispositif suivant l'invention.

Les figures 6 a et 6b représentent un dispositif réalisé à partir d'un treillis à structure fibrillaire ancré à la surface d'un élastomère.

La figure 7 représente un dispositif comportant des trous inclinés permettant son laçage avec des éléments de suture.

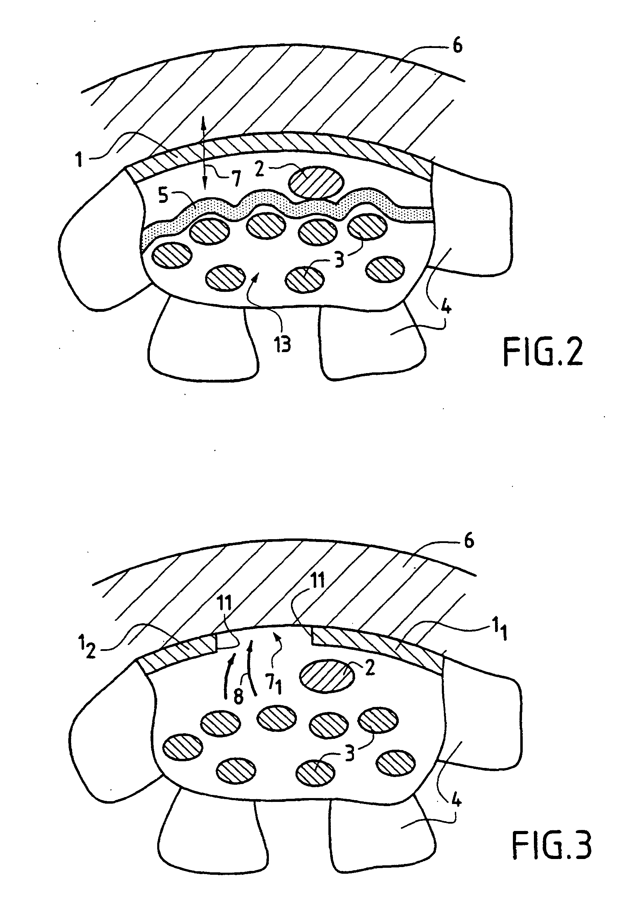

Suivant les figures 1 et 2, les os du poignet ou carpe 4, déterminent avec le ligament 1 annulaire antérieur du carpe, une zone canalaire 13 dans laquelle passent le nerf médian 2 et les tendons fléchisseurs 3.

Les tendons fléchisseurs 3 sont protégés par une gaine digito-carpienne interne 14. La zone canalaire 13 est entourée par les différents os du poignet 4, eux-mêmes entourés par les tendons extenseurs du poignet 15.

Une inflammation du tissu synovial qui entoure les tendons 3 peut provoquer une synovite 5 (des fléchisseurs) qui, en se développant, congestionne lesdits tendons 3 et comprime le nerf médian 2. Pour décomprimer cette zone, on peut alors à partir d'une ouverture dans la peau 6 située au-dessus couper 7 ledit ligament 1 pour, d'une part augmenter le volume de la zone canalaire 13, et d'autre part enlever la synoviale 5 par une telle intervention directe.

Après ladite intervention, les berges 1₁ et 1₂ du ligament, en restant ouvertes et flottantes, peuvent permettre la sortie 8 des tendons 3 et même éventuellement du nerf 2, à travers l'ouverture 7₁ ainsi laissée béante, tel que représenté sur la figure 3.

Pour éviter cet inconvénient ainsi que ceux rappelés précédemment, le dispositif de protection des nerfs 2 et/ou tendons 3 suivant l'invention comprend au moins une plaque 10 dont la forme et les dimensions notamment la largeur, sont compatibles avec la distance séparant les bords 11 des berges 1₁ et 1₂ dudit ligament 1 après coupure 7, et dont une face 10₂ comporte une surface de glissement, et l'autre face 10₁ une surface d'adhérence.

Pour permettre son insertion entre les deux berges 1₁ et 1₂ ligamentaires et reconstituer la continuité du ligament 1, les dimensions de ladite plaque 10 correspondent de préférence au plus juste avec celles de l'ouverture 7₁ du ligament 1, et son épaisseur est quasi égale à celle de ce ligament 1.

Compte tenu de l'ouverture qui, a priori, est faite suivant une ligne droite, ladite plaque 10 est alors de forme rectangulaire et, pour assurer une meilleure continuité dans la forme du ligament et pour la reconstitution du volume du canal carpien 13, ladite plaque 10 est de forme bombée avec deux côtés curvilignes parallèles, dans la direction transversale aux côtés correspondant aux bords 11 coupés du ligament 1, lui donnant ainsi une forme en "tuile".

De façon appropriée, ladite plaque est de forme tuilée du type calotte cylindrique avec une longueur des deux côtés rectilignes parallèles de 10 à 25 mm, et une courbure des deux côtés transversaux curvilignes parallèles, en arc de cercle ou de forme ovoïde, ayant un rayon de courbure de 5 à 10 mm, notamment de 7 mm, et des longueurs de corde correspondant aux dites courbures de 5 à 15 mm. Plus particulièrement, on réalise des plaques de longueur de 12 et 15 mm et de largeur 5, 6,7 et 8 mm.

Des plaques de ces dimensions (calottes cylindriques) peuvent s'adapter sur tous les types de patients et tous types d'incision, étant entendu que même pour des incisions plus importantes une plaque plus petite que l'incision permet néanmoins de remplir la fonction de protection recherchée.

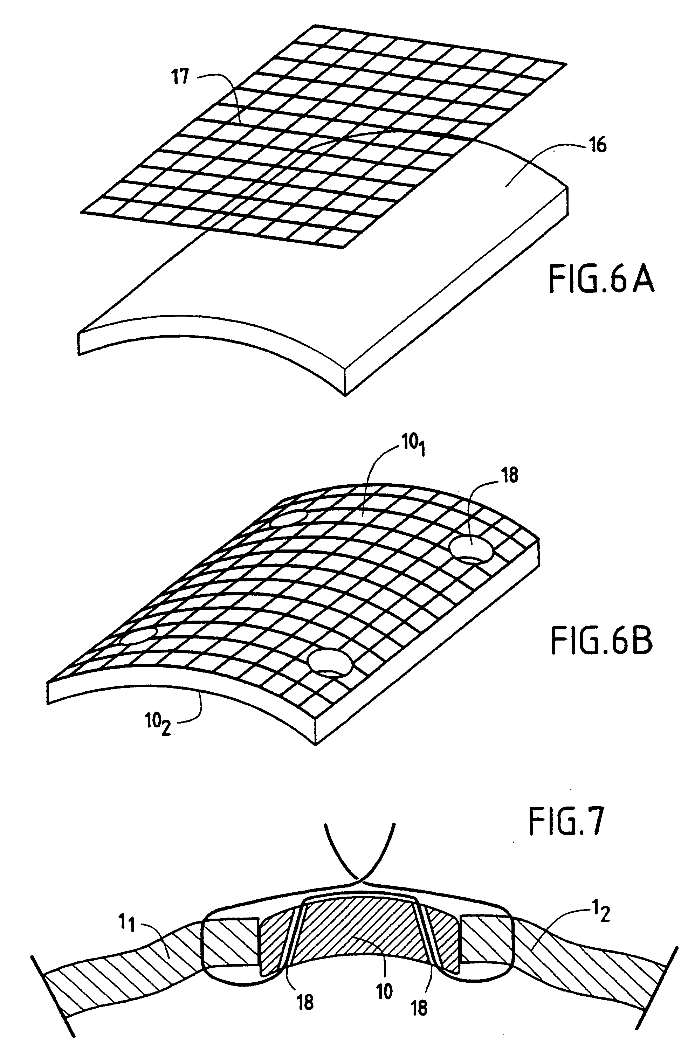

Pour constituer lesdites surfaces de glissement et d'adhérence représentées sur les figures 6, on a utilisé des matériaux non résorbables, comme respectivement le silicone pour la surface de glissement et des polymères biocompatibles sous forme de structure fibrillaire, tels que des fibres de polyester tèrèphtalate pour la surface d'adhérence.

Dans ce mode de réalisation des figures 6, ladite plaque est réalisée à partir d'une couche de silicone 16, avec ancré à sa surface sur une face qui constituera ainsi la surface d'adhérence un treillis 17 de fibres de polyester, notamment de PET, dont la structure fibrillaire présente une porosité caractérisée par une taille moyenne des pores comprise entre 50 et 600 microns. Le treillis de fibres de polymères polyester est appliqué à la surface de la plaqué de silicone en formation et se trouve ancré à sa surface après polymérisation complète du silicone.

Ce n'est pas la macro-texture ainsi créée à la surface du silicone sur une de ses faces qui confère des propriétés d'adhérence à ladite face, mais la rugosité ou la microporosité du matériau à structure fibrillaire constituant ledit treillis.

Dans ce mode de réalisation des figures 6, on a fabriqué une plaque avec une épaisseur de silicone de 1,5 mm recouverte d'un treillis de fibres de polyester de 0,5 mm d'épaisseur, ladite plaque comprenant 6% de polyester et 94% de silicone.

La plaque des figures 6 est obtenue à partir d'un treillis de PET. Les tissus en multi-filaments de fibres de polyester à mailles ajourées, tel qu'un tricot 16, sont connus de l'homme de l'Art. Ils sont utilisés en chirurgie sous forme d'un tricot à mailles ajourées réalisé en polyéthylène téréphtalate (PET). Ces tricots à mailles sont souples et sont utilisés tels quels comme implants souples pour la réfection de parois. Dans certains cas, les fils de polyester sont imprégnés de silicone, mais la structure en tricot ajouré est conservée. Cependant, la rugosité de surface liée à la microporosité de la structure fibrillaire n'est pas conservée.

Dans le mode de réalisation selon l'invention, les fils de polyester ne sont pas imprégnés de silicone en totalité et conservent une micropororité sur une de leurs faces non recouverte de silicone.

En effet, une plaque selon les figures 6, peut être obtenue comme suit : on dispose le treillis de fibres de PET au fond d'un moule de forme bombée correspondant à la forme voulue pour ladite plaque. On injecte le composé élastomère, notamment le silicone, sous basse pression de manière à remplir le moule sans enrober complètement le treillis de PET. L'enrobage partiel du treillis permet de conserver la face en regard du moule non recouverte de silicone, tout en ancrant ledit treillis à la surface du silicone, après polymérisation de ce dernier.

Ladite plaque 10 peut aussi être réalisée également en un seul matériau dont on traitera les deux faces, supérieure orientée vers la peau 6, et inférieure orientée vers la zone canalaire 13, pour obtenir respectivement les surfaces d'adhérence et de glissement voulues.

Si on utilise une plaque en métal comme mentionné précédemment, la surface d'adhérence peut être obtenue en traitant la surface métallique de manière à lui conférer une micro-texture ou une rugosité, notamment avec une valeur de Rt d'au moins 50 microns, de préférence au moins 100 microns.

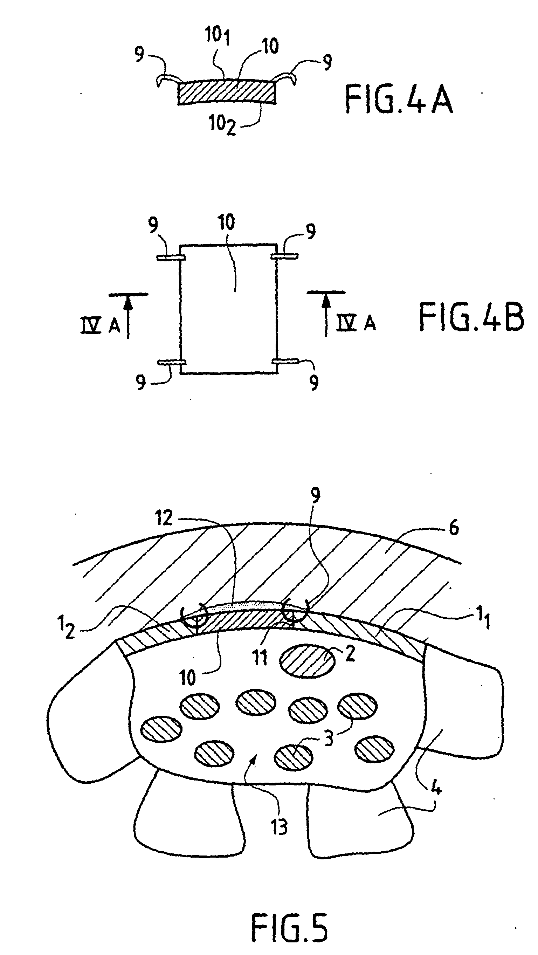

Pour assurer la fixation de la plaque 10 entre les berges 11 du ligament coupé, cette plaque peut comprendre soit au moins quatre trous 18 perpendiculaires aux surfaces de glissement et d'adhérence aptes à en assurer la suture 12 avec les bords 11 du ligament 1, tel que représenté sur la figure 6b, soit au moins quatre crochets 9 aptes à être directement ancrés dans les bords 11 du ligament 1, tel que représenté sur les figures 4. cette plaque peut aussi comprendre au moins quatre trous 18 inclinés (non perpendiculaires) aux surfaces de glissement et d'adhérence. L'inclinaison permet de réduire les contraintes transmises par les éléments de suture qui peuvent ainsi être lacés comme représenté figure 7.

L'inclinaison peut être variable suivant l'épaisseur et la largeur de la plaque. L'angle de l'inclinaison est tel que l'extrémité inférieure des trous est plus proche du bord latéral voisin de la plaque que l'extrémité supérieure.

## Revendications

1. Dispositif de protection des nerfs (2) et/ou tendons (3) situés dans une zone canalaire (13) du corps humain, normalement fermée par un ligament (1) et qui a été ouverte lors d'une intervention chirurgicale, **caractérisé en ce qu'**il comprend au moins une plaque rigide ou semi-rigide (10) destinée à être insérée entre les bords (11) dudit ligament (1) après coupure (7) et dont une face (10₂) comporte une surface de glissement et l'autre face (10₁) une surface d'adhérence.

2. Dispositif de protection selon la revendication 1, **caractérisé en ce que** ladite plaque présente une forme bombée avec une dite surface de glissement sur sa face concave et une dite surface d'adhérence sur sa face convexe.

3. Dispositif de protection selon l'une quelconque des revendications 1 à 2, **caractérisé en ce que** ladite plaque (10) est d'épaisseur quasi égale à celle dudit ligament (1) à l'endroit de ladite intervention.

4. Dispositif de protection selon l'une quelconque des revendications 1 à 3, **caractérisé en ce que** les dimensions de ladite plaque (10) correspondent avec celles de l'ouverture (7) du ligament (1), la longueur de la plaque étant au plus égale à celle de la coupure.

5. Dispositif de protection selon l'une quelconque des revendications 1 à 4, **caractérisé en ce que** ladite plaque (10) est de forme bombée avec au moins deux côtés curvilignes parallèles dans la direction transversale aux côtés correspondant aux bords (11) coupés du ligament (1).

6. Dispositif de protection selon l'une quelconque des revendications 1 à 5, **caractérisé en ce que** ladite plaque (10) est de forme tuilée correspondant à une section de surface cylindrique comprenant deux côtés rectilignes parallèles et deux côtés curvilignes parallèles.

7. Dispositif de protection selon l'une quelconque des revendications 1 à 6, **caractérisé en ce que** ladite plaque est de forme bombée avec quatre côtés curvilignes parallèles deux à deux.

8. Dispositif de protection selon l'une quelconque des revendications 1 à 7, **caractérisé en ce que** les quatre angles de ladite plaque définissent un rectangle dont la longueur est inférieure à la taille de la coupe et le rapport largeur/longueur est de 1/4 à 3/4, de préférence 1/3 à 2/3.

9. Dispositif de protection selon l'une quelconque des revendications 1 à 8, **caractérisé en ce que** ladite plaque est réalisée en matériau(x) résorbable(s).

10. Dispositif de protection selon l'une quelconque des revendications 1 à 9, **caractérisé en ce que** ladite plaque (10) est fabriquée en deux couches de biomatériaux assemblées entre elles et correspondant chacune et respectivement à la surface de glissement (10₂) et à la surface d'adhérence (10₁).

11. Dispositif de protection selon l'une quelconque des revendications 1 à 10, **caractérisé en ce que** ladite surface de glissement est réalisée avec un composé élastomère biocompatible, et ladite surface d'adhérence comprend un matériau polymère biocompatible à structure microporeuse.

12. Dispositif de protection selon l'une quelconque des revendications 1 à 11, **caractérisé en ce que** la rugosité Ra de la surface de glissement est inférieure à 3,2 microns, de préférence 0,8 microns.

13. Dispositif de protection selon l'une quelconque des revendications 1 à 12, **caractérisé en ce que** la rugosité Rt de la surface d'adhérence est supérieure à 50 microns.

14. Dispositif de protection selon l'une quelconque des revendications 1 à 13, **caractérisé en ce que** la rugosité Ra de la surface de glissement est inférieure à 3,2 microns, de préférence inférieure à 0,8 microns, et ladite surface d'adhérence comprend un matériau à structure microporeuse comportant des pores dont la taille moyenne est de 50 à 600 microns.

15. Dispositif de protection selon l'une quelconque des revendications 11 à 14, **caractérisé en ce que** ladite surface de glissement (10₂) est constituée de silicone et ladite surface d'adhérence (10₂) comprend du polyester.

16. Dispositif de protection selon l'une quelconque des revendications 10 à 15, **caractérisé en ce que** ladite plaque (10) est constituée de silicone avec un treillis de fibres de polyester ancré sur l'une de ses faces.

17. Dispositif de protection selon l'une quelconque des revendications 10 à 16, **caractérisé en ce que** la plaque comprend 5 à 10% en poids de matériau microporeux et 60 à 95% en poids de composé élastomère, le total des deux constituants faisant 100%.

18. Dispositif de protection selon l'une quelconque des revendications 9 et 12 à 13, **caractérisé en ce que** ladite plaque est réalisée en métal ou matériau composite dont une face est lisse et correspond à ladite surface de glissement, et l'autre face présente une structure rugueuse correspondant à ladite surface d'adhérence.

19. Dispositif de protection selon l'une quelconque des revendications 1 à 18, **caractérisé en ce que** ladite plaque (10) comprend au moins quatre trous aptes à en assurer la suture (12) avec les bords (11) du ligament (1).

20. Dispositif de protection selon l'une quelconque des revendications 1 à 18, **caractérisé en ce que** ladite plaque (10) comporte au moins quatre crochets (9) aptes à être ancrés dans les bords (11) du ligament (1).

21. Dispositif de protection selon la revendication 19, **caractérisé en ce que** les trous sont inclinés de manière à permettre le laçage des éléments de suture.

## Patentansprüche

1. Vorrichtung zum Schutz von Nerven (2) und/oder Sehnen (3), die sich in einer Tunnelzone (13) des menschlichen Körpers befinden, die normalerweise durch ein Band (1) verschlossen ist und die durch einen chirurgischen Eingriff eröffnet wurde, **dadurch gekennzeichnet, dass** sie mindestens eine steife oder semi-steife Platte (10) umfasst, die dazu bestimmt ist, zwischen die Ränder (11) des Bandes (1) nach einem Schnitt (7) eingesetzt zu werden, und bei der eine Seite (10₂) eine Gleitseite und die andere Seite (10₁) eine Haftseite trägt.

2. Schutzvorrichtung nach Anspruch 1, **dadurch gekennzeichnet, dass** die Platte eine gewölbte Form mit einer Gleitoberfläche auf ihrer konkaven Seite und eine Haftoberfläche auf ihrer konvexen Seite aufweist.

3. Schutzvorrichtung nach einem der Ansprüche 1 bis 2, **dadurch gekennzeichnet, dass** die Platte (10) praktisch die gleiche Dicke hat wie das Band (1) an der Stelle des Eingriffs.

4. Schutzvorrichtung nach einem der Ansprüche 1 bis 3, **dadurch gekennzeichnet, dass** die Abmessungen der Platte (10) denjenigen der Öffnung (7) des Bandes (1) entsprechen, wobei die Länge der Platte höchstens gleich derjenigen des Schnitts ist.

5. Schutzvorrichtung nach einem der Ansprüche 1 bis 4, **dadurch gekennzeichnet, dass** die Platte (10) eine gewölbte Form hat, wobei mindestens zwei in Querrichtung zu den Seiten parallele gekrümmte Seiten den geschnittenen Rändern (11) des Bandes (1) entsprechen.

6. Schutzvorrichtung nach einem der Ansprüche 1 bis 5, **dadurch gekennzeichnet, dass** die Platte (10) eine dachziegelartige Form hat, die einem Abschnitt der zylindrischen Oberfläche entspricht, die zwei parallele gerade Seiten und zwei parallele gekrümmte Seiten umfasst.

7. Schutzvorrichtung nach einem der Ansprüche 1 bis 6, **dadurch gekennzeichnet, dass** die Platte eine gewölbte Form mit vier gekrümmten Seiten hat, von denen jeweils zwei parallel sind.

8. Schutzvorrichtung nach einem der Ansprüche 1 bis 7, **dadurch gekennzeichnet, dass** die vier Winkel der Platte ein Rechteck definieren, dessen Länge kleiner ist als die Größe des Schnitts und dessen Verhältnis Breite/Länge 1/4 bis 3/4, vorzugsweise 1/3 bis 2/3 beträgt.

9. Schutzvorrichtung nach einem der Ansprüche 1 bis 8, **dadurch gekennzeichnet, dass** die Platte aus resorbierbare(m/n) Material(ien) hergestellt wird.

10. Schutzvorrichtung nach einem der Ansprüche 1 bis 9, **dadurch gekennzeichnet, dass** die Platte (10) aus zwei Schichten von Biomaterialien hergestellt wird, die miteinander vereinigt sind und von denen jede der Gleitoberfläche (10₂) bzw. der Haftoberfläche (10₁) entspricht.

11. Schutzvorrichtung nach einem der Ansprüche 1 bis 10, **dadurch gekennzeichnet, dass** die Gleitoberfläche aus einer biokompatiblen Elastomerverbindung hergestellt wird und die Haftoberfläche ein biokompatibles Polymermaterial mit mikroporöser Struktur umfasst.

12. Schutzvorrichtung nach einem der Ansprüche 1 bis 11, **dadurch gekennzeichnet, dass** die Rauheit Ra der Gleitoberfläche kleiner als 3,2 Mikron, vorzugsweise 0,8 Mikron, ist.

13. Schutzvorrichtung nach einem der Ansprüche 1 bis 12, **dadurch gekennzeichnet, dass** die Rauheit Rt der Haftoberfläche größer als 50 Mikron ist.

14. Schutzvorrichtung nach einem der Ansprüche 1 bis 13, **dadurch gekennzeichnet, dass** die Rauheit Ra der Gleitoberfläche kleiner als 3,2 Mikron, vorzugsweise kleiner als 0,8 Mikron, ist und die Haftoberfläche ein Material mit mikroporöser Struktur umfasst, das Poren mit einem mittleren Durchmesser von 50 bis 600 Mikron trägt.

15. Schutzvorrichtung nach einem der Ansprüche 11 bis 14, **dadurch gekennzeichnet, dass** die Gleitoberfläche (10₂) aus Silikon besteht und die Haftoberfläche (10₁) Polyester umfasst.

16. Schutzvorrichtung nach einem der Ansprüche 10 bis 15, **dadurch gekennzeichnet, dass** die Platte (10) aus Silikon besteht, wobei ein Geflecht aus Polyesterfasern auf einer ihrer Seiten verankert ist.

17. Schutzvorrichtung nach einem der Ansprüche 10 bis 16, **dadurch gekennzeichnet, dass** die Platte 5 bis 10 Gew.-% mikroporöses Material und 60 bis 95 Gew.-% Elastomerverbindung umfasst, wobei diese beiden Bestandteile insgesamt 100% ausmachen.

18. Schutzvorrichtung nach einem der Ansprüche 9 und 12 bis 13, **dadurch gekennzeichnet, dass** die Platte aus Metall oder Verbundmaterial hergestellt ist, dessen eine Seite glatt ist und der Gleitoberfläche entspricht und dessen andere Seite eine raue Struktur aufweist, die der Haftoberfläche entspricht.

19. Schutzvorrichtung nach einem der Ansprüche 1 bis 18, **dadurch gekennzeichnet, dass** die Platte (10) mindestens vier Löcher umfasst, die dazu bestimmt sind, das Vernähen (12) mit den Rändern (11) des Bandes (1) zu gewährleisten.

20. Schutzvorrichtung nach einem der Ansprüche 1 bis 18, **dadurch gekennzeichnet, dass** die Platte (10) mindestens vier Haken (9) trägt, die dazu bestimmt sind, an den Rändern (11) des Bandes (1) verankert zu werden.

21. Schutzvorrichtung nach Anspruch 19, **dadurch gekennzeichnet, dass** die Löcher derart geneigt sind, dass sie das Zusammennähen der Nahtelemente gestatten.

## Claims

1. Device for protecting nerves (2) and/or tendons (3) located in a tunnel zone (13) of the human body which is normally closed by a ligament (1) and has been opened during a surgical procedure, **characterized in that** said device comprises at least one rigid or semirigid plate (10) which is intended to be inserted between the edges (11) of said ligament (1) after cutting (7) and of which one face (10₂) comprises a sliding surface and the other face (10₁) comprises an adhering surface.

2. Device for protection according to Claim 1, **characterized in that** said plate has a bulged shape with one said sliding surface on its concave face and one said adhering surface on its convex face.

3. Device for protection according to either one of Claims 1 and 2, **characterized in that** said plate (10) has a thickness almost equal to that of said ligament (1) at the site of said procedure.

4. Device for protection according to any one of Claims 1 to 3, **characterized in that** the dimensions of said plate (10) correspond to those of the opening (7) in the ligament (1), the length of the plate being at most equal to that of the cut.

5. Device for protection according to any one of Claims 1 to 4, **characterized in that** said plate (10) has a bulged shape with at least two parallel curvilinear sides in the direction transverse to the sides corresponding to the cut edges (11) of the ligament (1).

6. Device for protection according to any one of Claims 1 to 5, **characterized in that** said plate (10) has a tile shape corresponding to a section of cylindrical surface comprising two parallel rectilinear sides and two parallel curvilinear sides.

7. Device for protection according to any one of Claims 1 to 6, **characterized in that** said plate has a bulged shape with four parallel curvilinear sides arranged in pairs.

8. Device for protection according to any one of Claims 1 to 7, **characterized in that** the four corners of said plate define a rectangle of which the length is less than the size of the cut and of which the width/length ratio is from 1/4 to 3/4, preferably 1/3 to 2/3.

9. Device for protection according to any one of Claims 1 to 8, **characterized in that** said plate is made of absorbable material(s).

10. Device for protection according to any one of Claims 1 to 9, **characterized in that** said plate (10) is made up of two layers of biomaterial joined to each other and each corresponding respectively to the sliding surface (10₂) and to the adhering surface (10₁).

11. Device for protection according to any one of Claims 1 to 10, **characterized in that** said sliding surface is made with a biocompatible elastomeric compound, and said adhering surface comprises a biocompatible polymer material of microporous structure.

12. Device for protection according to any one of Claims 1 to 11, **characterized in that** the roughness Ra of the sliding surface is less than 3.2 microns, preferably 0.8 micron.

13. Device for protection according to any one of Claims 1 to 12, **characterized in that** the roughness Rt of the adhering surface is greater than 50 microns.

14. Device for protection according to any one of Claims 1 to 13, **characterized in that** the roughness Ra of the sliding surface is less than 3.2 microns, preferably less than 0.8 micron, and said adhering surface comprises a material of microporous structure having pores with an average size of from 50 to 600 microns.

15. Device for protection according to any one of Claims 11 to 14, **characterized in that** said sliding surface (10₂) is made of silicone and said adhering surface (10₂) comprises polyester.

16. Device for protection according to any one of Claims 10 to 15, **characterized in that** said plate (10) is made of silicone with a lattice of polyester fibres anchored on one of its faces.

17. Device for protection according to any one of Claims 10 to 16, **characterized in that** the plate comprises 5 to 10% by weight of microporous material and 60 to 95% by weight of elastomeric compound, the total of the two constituents amounting to 100%.

18. Device for protection according to any one of Claims 9 and 12 to 13, **characterized in that** said plate is made of metal or composite material of which one face is smooth and corresponds to said sliding surface, and of which the other face has a rough structure corresponding to said adhering surface.

19. Device for protection according to any one of Claims 1 to 18, **characterized in that** said plate (10) comprises at least four holes to ensure suturing (12) thereof to the edges (11) of the ligament (1).

20. Device for protection according to any one of Claims 1 to 18, **characterized in that** said plate (10) comprises at least four hooks (9) which can be anchored in the edges (11) of the ligament (1).

21. Device for protection according to Claim 19, **characterized in that** the holes are inclined in such a way as to permit the lacing of the suture elements.
